# EUROPÄISCHE PATENTSCHRIFT

(11) **EP 3 727 125 B1**
(45) Veröffentlichungstag und Bekanntmachung des Hinweises auf die Patenterteilung: **26.07.2023**
(21) Anmeldenummer: 18857387.7
(22) Anmeldetag: 19.12.2018
(51) Int. Cl.: A61B 1/00, A61B 1/018

(54) **ENDOSKOP**
ENDOSCOPE
ENDOSCOPE

(30) Priorität: 21.12.2017 DE 102017130905
(43) Veröffentlichungstag der Anmeldung: 28.10.2020
(73) Patentinhaber: Blazejewski Medi-Tech GmbH, 79350 Sexau (DE)
(72) Erfinder: BLAZEJEWSKI, Reinhold, 79261 Gutach (DE)
(74) Vertreter: Geitz Truckenmüller Lucht Christ
(86) Internationale Anmeldenummer: PCT/DE2018/101032
(87) Internationale Veröffentlichungsnummer: WO 2019/120384

(56) Entgegenhaltungen:
- US-A- 5 213 093
- US-A1- 2008 045 787
- US-B2- 9 119 531

## Beschreibung

Die Erfindung geht aus von einem Endoskop mit einem Schaft, einem Grundkörper, in welchem der Schaft mit seinem proximalen Ende aufgenommen ist, einem Bildleiter und einem Arbeitskanal, welche sich vom distalen bis zum proximalen Ende durch den Schaft erstrecken und im Grundkörper fortgesetzt sind.

Endoskope werden sowohl im technischen als auch im medizinischen Bereich eingesetzt. Sie dienen der Untersuchung von Strukturen an der Oberfläche oder in schwer zugänglichen Hohlräumen, Kanälen oder Vertiefungen. Diese Strukturen sind häufig mit bloßem Auge nicht aufzulösen. Im medizinischen Bereich werden Endoskope in der minimalinvasiven Chirurgie zu Untersuchungszwecken oder in Kombination mit chirurgischen Instrumenten für Operationen unter Sichtkontrolle eingesetzt. Ein Beleuchtungssystem kann dazu dienen, die zu untersuchende Struktur zu beleuchten. Das durch eine externe Lichtquelle erzeugte Licht wird üblicherweise über Lichtleiter, insbesondere Lichtleitfasern, an die zu untersuchende Struktur herangeführt. Der Lichtleiter kann in das Endoskop integriert sein. Ein Bildgebungssystem dient dazu, die Information, welche in dem von der Struktur reflektierten Licht enthalten ist, als Bild aufzunehmen. Als Kamera oder Bildsensor dient häufig ein Bildwandlerchip, beispielsweise CMOS oder CCD. Der Bildsensor, auch Bildgeber genannt, wandelt die optischen Signale in elektrische Signale um, welche anschließend auf einem Bildschirm oder einem Monitor optisch sichtbar gemacht werden. Alternativ oder kumulativ kann das Endoskop mit einem Okular ausgestattet sein.

Bei bekannten Endoskopen sind der oder die Bildgeber am proximalen oder am distalen Ende angeordnet. Das Endoskop umfasst einen flexiblen oder starren Schaft, der als länglicher Hohlkörper ausgebildet ist, und einen Grundkörper, in den der Schaft mit seinem proximalen Ende aufgenommen ist. Der Schaft wird in einen zu untersuchenden Hohlraum eingeführt. Der Schaft erstreckt sich entlang einer in seiner Längsrichtung verlaufenden Längsachse. Der äußere Querschnitt des Schaftes ist bevorzugt rund, insbesondere kreisrund. Der Grundkörper dient der Handhabung des Endoskops. Das von einer zu untersuchenden Struktur reflektierte Licht wird am distalen Ende des Schaftes über ein Objektiv eingekoppelt und über ein optisches Bildleitungssystem mit optischen Komponenten wie Linsen und Prismen oder durch eine Faseroptik dem Bildgeber zugeführt. Der Bildgeber wird auch als Bildsensor bezeichnet. Der Bildleiter umfasst das Objektiv am distalen Ende des Schaftes, das optische Bildleitungssystem und den Bildgeber. Ist der Bildsensor am distalen Ende des Schaftes angeordnet, so werden die von dem Bildsensor aus dem reflektierten Licht erzeugten elektrischen Signale mittels einer Signalleitung an das proximale Ende des Schaftes und in den Grundkörper geleitet. Diese Signalleitung gehört ebenfalls zum Bildleiter. Die von dem Bildsensor dabei aufgenommenen Bilder werden durch eine Bildbearbeitungseinrichtung bearbeitet und auf einem Sichtgerät für den Benutzer sichtbar dargestellt.

Neben einem Bildleiter und einem Lichtleiter weisen bekannte Endoskope einen Arbeitskanal auf. Bildleiter und Arbeitskanal erstrecken sich vom Grundkörper durch den Schaft hindurch bis zu dessen distalem Ende. In den Arbeitskanal wird ein Instrument eingeführt. Der Arbeitskanal weist an seinem proximalen Ende an dem Grundkörper eine Öffnung auf, durch die das Instrument eingeführt wird. Das Instrument wird durch den Arbeitskanal bis an das distale Ende des Schaftes bewegt um dort eine mithilfe des Bildleiters betrachtete Struktur zu bearbeiten. Zu derartigen Instrumenten zählen beispielsweise eine Zange, ein Greifer oder eine Schere. Sie dienen zum Greifen, Halten, Schneiden, Stanzen, Quetschen oder sonstigen Manipulieren der Struktur.

Soll das Endoskop der Untersuchung von Strukturen in kleinen Hohlräumen dienen, muss der Durchmesser des Schaftes insbesondere am distalen Ende möglichst klein sein. Der Durchmesser des Schaftes muss kleiner sein als der des Hohlraums, in den das Endoskop eingeführt werden soll. Dies wiederum bedeutet, dass der Querschnitt des in dem Schaft verlaufenden Arbeitskanals entsprechend noch kleiner sein muss, was zur Folge hat, dass die in den Arbeitskanal eingesetzten Instrumente ebenfalls einen kleinen Durchmesser aufweisen müssen. Hinzu kommt, dass Instrumente wie Zangen, Geifer oder Scheren Stücke aus der bearbeiteten Struktur entfernen und aufnehmen können. Diese Stücke werden in dem Instrument eingeklemmt oder eingespannt und zusammen mit dem Instrument aus dem Arbeitskanal herausgeführt. Ist ein derartiges Stück an dem Instrument eingeklemmt, so kann der Durchmesser des Instruments entsprechend vergrößert sein.

US 2008/0045787 A1 offenbart ein Endoskop entsprechend der Präambel von Anspruch 1.

Der Erfindung liegt die Aufgabe zugrunde, ein Endoskop mit Arbeitskanal für ein Instrument zur Verfügung zu stellen, bei dem der Schaft zur Einführung in Hohlräume einen möglichst kleinen Durchmesser aufweist und der in dem Schaft verlaufende Arbeitskanal trotzdem einen für die Handhabung von Instrumenten notwendigen Querschnitt besitzt, der insbesondere auch dann das Zurückziehen des Instrumentes durch den Arbeitskanal ermöglicht, wenn das Instrument ein Stück einer zu untersuchenden Struktur entfernt und derart einspannt, dass das Stück zusammen mit dem Instrument aus dem Arbeitskanal herausgeführt wird, während der Schaft des Endoskops in dem Hohlraum verbleibt.

Diese Aufgabe wird durch ein Endoskop mit den Merkmalen des Anspruchs 1 gelöst. Es zeichnet sich dadurch aus, dass der Arbeitskanal an seinem proximalen Ende einen kreisrunden Querschnitt und an seinem distalen Ende einen von kreisrund verschiedenen Querschnitt aufweist. Das proximale Ende des Arbeitskanals befindet sich am oder im Grundkörper. Das Endoskop wird mit dem distalen Ende voraus in einen zu untersuchenden Hohlraum eingeführt. Der Grundkörper bleibt stets außerhalb des Hohlraums. Daher steht für den Abschnitt des Arbeitskanals, der im Grundkörper verläuft, ausreichend Platz zur Verfügung. Der Arbeitskanal weist am proximalen Ende einen kreisrunden Querschnitt auf, was das Einführen eines Instruments in den Arbeitskanal an seinem proximalen Ende erleichtert. Darüber hinaus lassen sich entsprechende Hohlkörper leicht und vergleichsweise kostengünstig herstellen. An seinem distalen Ende weist der Querschnitt des Arbeitskanals eine von kreisrund verschiedene Form auf. Aufgrund dieser Form des Arbeitskanals am distalen Ende, steht in dem Schaft selbst bei kleinen Schaftdurchmessern innerhalb des Schaftes neben dem Arbeitskanal ausreichend Platz für einen Bildleiter und bei Bedarf noch zusätzlich für einen oder mehrere Lichtleiter und gegebenenfalls für einen oder mehrere Spülkanäle zur Verfügung. Der Schaft selbst kann dabei eine für das Einführen in einen Hohlraum und das Ausrichten in einem Hohlraum kreisrunden äußeren Querschnitt aufweisen.

In bevorzugter Weise weist der Arbeitskanal in dem Abschnitt des Schaftes, der in einen Hohlraum einführbar ist, einen Querschnitt mit konstanter Querschnittsfläche auf. Dies bedeutet, dass sich die Form und Größe der Querschnittsfläche des Arbeitskanals in dem betreffenden Abschnitt des Schaftes nicht ändert. Dies hat zur Folge, dass auch der Schaft eine konstante Querschnittsfläche in diesem Abschnitt aufweist.

Der von einer Kreisform abweichende Querschnitt des Arbeitskanals weist einen größten Durchmesser und einen kleinsten Durchmesser auf. Ein Instrument, das vor der Bearbeitung einer Struktur beim Einführen in den Arbeitskanal einen kleineren Durchmesser als der kleinste Durchmesser des von einer Kreisform abweichenden Querschnitt des Arbeitskanals aufweist, kann nach der Aufnahme eines Stücks so gedreht werden, dass ihm beim Herausführen aus dem Arbeitskanal der größte Durchmesser des betreffenden Abschnitts des Arbeitskanals zur Verfügung steht. Dadurch wird gewährleistet, dass das Instrument auch dann aus dem Arbeitskanal herausgezogen werden kann, wenn das Instrument ein Stück der zu bearbeitenden Struktur aufgenommen hat. Es kann vermieden werden, dass in diesem Fall das gesamte Endoskop aus dem Hohlraum entfernt werden muss. Dies ist insbesondere bei medizinischen Anwendungen von erheblichem Vorteil, da durch das wiederholte Einführen und Herausziehen des gesamten Schaftes aus einem Hohlraum die Gefahr von Verletzungen und Infektionen erhöht wird.

Zwischen dem proximalen Ende mit kreisrundem Querschnitt und dem distalen Ende mit von einer Kreisform abweichendem Querschnitt weist der Arbeitskanal einen Übergangsbereich auf, in dem der kreisrunde Querschnitt in den von kreisrund abweichenden Querschnitt übergeht. Vorteilhafterweise ändert sich in dem Übergangsbereich der Querschnitt stetig, so dass eine den Arbeitskanal begrenzende Wandung eine Rampe oder eine Art Trichter ausbildet, an dem ein Instrument bei Einführen in den Arbeitskanal entlang gleiten kann. Dadurch wird ein Verkanten vermieden. Alternativ dazu kann der Übergangsbereich eine oder mehrere Stufen ausbilden.

Damit weist das erfindungsgemäße Endoskop trotz eines Schaftes mit insbesondere am distalen Ende kleinem Durchmesser einen Arbeitskanal auf, der ein Instrument auch dann aufnehmen kann, wenn dieses einen etwas größeren Durchmesser als der kleinste Durchmesser des ovalen Abschnitts des Arbeitskanals besitzt. Dies gilt insbesondere dann, wenn das Instrument ein Stück der zu bearbeitenden Struktur aufgenommen hat, welches zusammen mit dem Instrument aus dem Arbeitskanal herausbewegt werden soll. Gleichzeitig weist der Arbeitskanal an seinem proximalen Ende einen kreisrunden Querschnitt auf, so dass das Einführen eines Instrumentes erleichtert ist. Das Instrument kann beim Einführen in den Arbeitskanal in beliebiger Weise gedreht werden.

Nach einer vorteilhaften Ausgestaltung der Erfindung ist die Querschnittsfläche des Arbeitskanals am proximalen Ende größer ist als am distalen Ende.

Nach einer weiteren vorteilhaften Ausgestaltung der Erfindung weist der Abschnitt des Arbeitskanals, welcher sich außerhalb des Grundkörpers in dem Schaft erstreckt, durchgängig einen von kreisrund abweichenden Querschnitt mit konstanter Querschnittsfläche auf. Als Folge davon weist auch der Schaft außerhalb des Grundkörpers durchgängig einen übereinstimmenden Querschnitt mit konstanter Querschnittsfläche auf. Dies erleichtert das Einführen des Schaftes in einen Hohlraum.

Nach einer weiteren vorteilhaften Ausgestaltung der Erfindung weist der Arbeitskanal zwischen dem Übergangsbereich und dem proximalen Ende durchgängig einen kreisrunden Querschnitt mit konstanter Querschnittsfläche auf.

Nach einer weiteren vorteilhaften Ausgestaltung der Erfindung ist der Übergangsbereich im Wesentlichen konisch oder kegelförmig.

Erfindungsgemäß befindet sich der Übergangsbereich im Grundkörper.

Nach einer weiteren vorteilhaften Ausgestaltung der Erfindung weist die von kreisrund verschiedene Querschnittsfläche des Arbeitskanals an seinem distalen Ende einen größten Durchmesser und einen kleinsten Durchmesser auf. Dabei ist der größte Durchmesser kleiner oder gleich wie der Durchmesser der kreisrunden Querschnittsfläche des Arbeitskanals an seinem proximalen Ende.

Nach einer weiteren vorteilhaften Ausgestaltung der Erfindung entspricht der Mittelpunkt der von kreisrund verschiedenen Querschnittsfläche des Arbeitskanals an seinem distalen Ende dem Schnittpunkt aus größtem Durchmesser und kleinstem Durchmesser.

Nach einer weiteren vorteilhaften Ausgestaltung der Erfindung weist der Arbeitskanal eine erste Arbeitskanal-Längsachse in seinem Abschnitt mit von kreisrund abweichenden Querschnitt auf, welche sich durch die Mittelpunkte der von kreisrund verschiedenen Querschnittsflächen erstreckt. Darüber hinaus weist der Arbeitskanal eine zweite Arbeitskanal-Längsachse in seinem Abschnitt mit kreisrundem Querschnitt auf, welche sich durch die Mittelpunkte der kreisrunden Querschnittsflächen erstreckt.

Nach einer weiteren vorteilhaften Ausgestaltung der Erfindung fällt die Verlängerung der ersten Arbeitskanal-Längsachse mit der zweiten Arbeitskanal-Längsachse zusammen.

Nach einer weiteren vorteilhaften Ausgestaltung der Erfindung ist die Verlängerung der ersten Arbeitskanal-Längsachse parallel zu der zweiten Arbeitskanal-Längsachse und weist zu der zweiten Arbeitskanal-Längsachse einen Abstand a auf, welcher von Null verschieden ist. Damit sind die beiden Arbeitskanal-Längsachsen seitlich versetzt zueinander.

Nach einer weiteren vorteilhaften Ausgestaltung der Erfindung ist der Abstand a kleiner oder gleich der Differenz aus dem halben Durchmesser der kreisrunden Querschnittsfläche des Arbeitskanals an seinem proximalen Ende und dem halben kleinsten Durchmesser der von kreisrund verschiedenen Querschnittsfläche des Arbeitskanals an seinem distalen Ende.

Nach einer weiteren vorteilhaften Ausgestaltung der Erfindung ist der von einer kreisrunden Form abweichenden Querschnitt rund. Der Querschnitt weist damit keine Ecken auf. Dies hat den Vorteil, dass ein in den Arbeitskanal eingeführtes Werkzeug beim Ein- und Ausführen und beim sonstigen Bewegen in dem Arbeitskanal nicht verkanten kann. Die Form des Querschnitts kann beispielsweise oval oder nierenförmig sein.

Nach einer weiteren vorteilhaften Ausgestaltung der Erfindung ist der von kreisrund verschiedene Querschnitt am distalen Ende des Arbeitskanals oval. Der Querschnitt ist damit rund aber nicht kreisrund. Die ovale Form zeichnet sich dadurch aus, dass sowohl der kleinste Durchmesser als auch der größte Durchmesser vollständig innerhalb der Querschnittsfläche verlaufen.

Weitere Vorteile und vorteilhafte Ausgestaltungen der Erfindung sind der nachfolgenden Beschreibung, der Zeichnung und den Ansprüchen entnehmbar.

### Zeichnung

In der Zeichnung ist ein Ausführungsbeispiel der Erfindung dargestellt. Es zeigen:
- Figur 1: Endoskop im Längsschnitt,
- Figur 2: Endoskop gemäß Figur 1 in einer Ansicht von oben,
- Figur 3: Endoskop gemäß Figur 1 in einer Ansicht von vorne,
- Figur 4: mit A in Figur 3 gekennzeichneter Ausschnitt aus Figur 3, welche nur die Aufsicht auf die distale Stirnseite des Schaftes zeigt,
- Figur 5: Arbeitskanal des Endoskops gemäß Figur 1 in einer Seitenansicht,
- Figur 6: Aufsicht auf den Arbeitskanal gemäß Figur 5 in der mit B gekennzeichneten Blickrichtung in Figur 5.

### Beschreibung des Ausführungsbeispiels

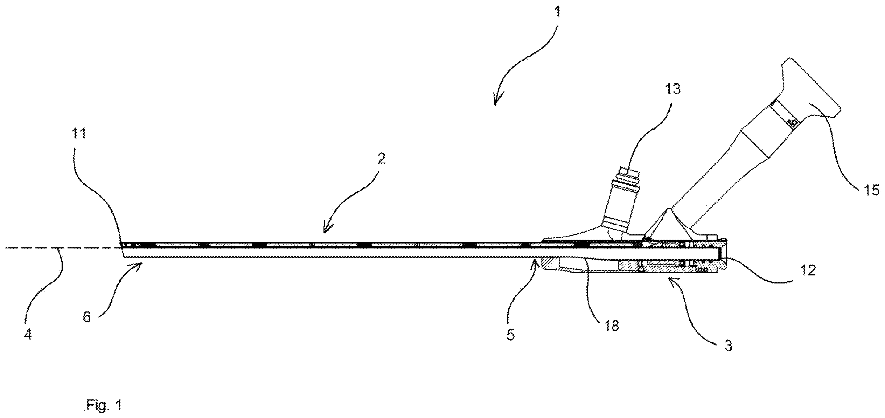

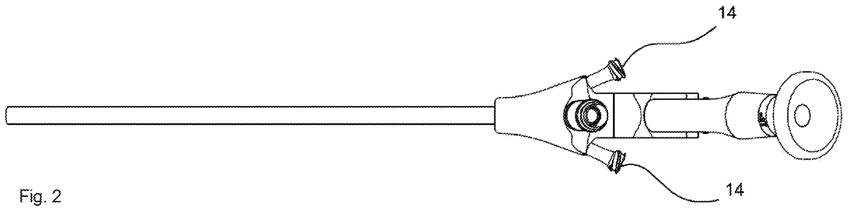

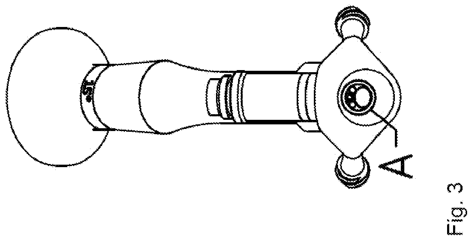

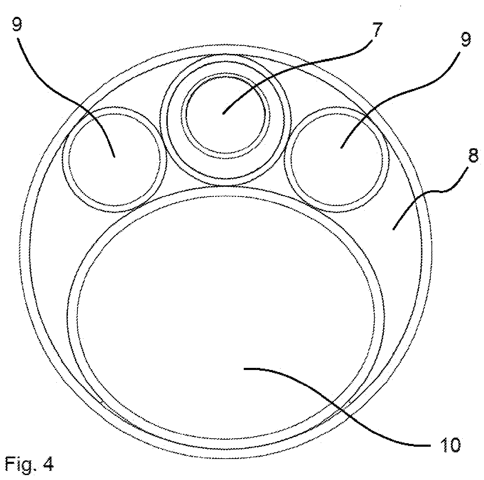

In den Figuren 1 bis 6 ist ein Ausführungsbeispiel eines Endoskops 1 dargestellt. Das Endoskop weist einen länglichen Schaft 2 und einen Grundkörper 3 auf. Der Schaft 2 erstreckt sich entlang einer Längsachse 4. Der Schaft weist ein dem Grundkörper 3 zugewandtes proximales Ende 5 und ein dem Grundkörper 3 abgewandtes distales Ende 6 auf. Mit seinem proximalen Ende 5 ist der Schaft 2 in dem Grundkörper 3 aufgenommen. Durch den Schaft 2 verlaufen ein Bildleiter 7, zwei Lichtleiter 8, zwei Spülkanäle 9 und ein Arbeitskanal 10. Bildleiter 7, Lichtleiter 8, Spülkanäle 9 und Arbeitskanal 10 münden an der Stirnseite des distalen Endes 6 des Schaftes 2. Dies ist besonders gut in Figur 4 erkennbar. Der Arbeitskanal 10 erstreckt sich vom distalen Ende 6 des Schaftes 2 bis an die dem Schaft 2 abgewandte Stirnseite des Grundkörpers 3. Das distale Ende 11 des Arbeitskanals 10 befindet sich an dem distalen Ende 6 des Schaftes 2. Das proximale Ende 12 des Arbeitskanals 10 befindet sich an der dem Schaft 2 abgewandten Stirnseite des Grundkörpers 3. An dem proximalen Ende 12 kann in den Arbeitskanal 10 ein in der Zeichnung nicht dargestelltes Instrument eingeführt und bis zum distalen Ende 11 des Arbeitskanals 10 vorgeschoben werden. Die Lichtleiter 8 münden an dem Grundkörper 3 in einen Lichtleiter-Anschluss 13, an den eine in der Zeichnung nicht dargestellte Lichtquelle über einen weiteren Lichtleiter gekoppelt werden kann. Die beiden Spülkanäle münden an dem Grundkörper 3 in zwei Spülkanal-Anschlüsse 14. Der Bildleiter 7 erstreckt sich in dem Grundkörper 3 bis zu einem Okular 15.

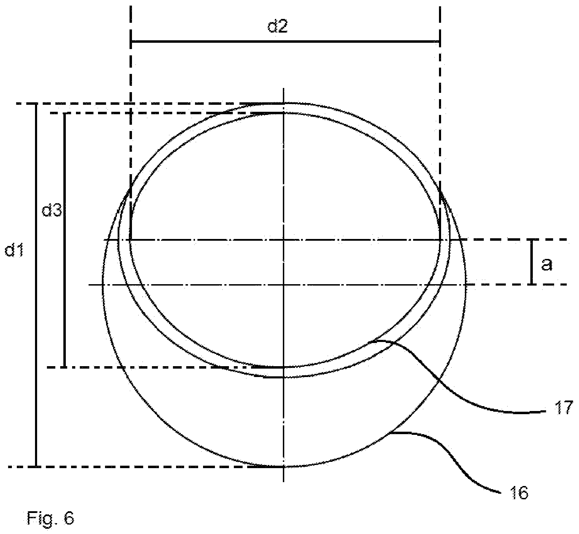

In Figur 1 ist der Arbeitskanal 10 im Längsschnitt dargestellt. Die Querschnittsflächen des Arbeitskanals 10 sind in Figur 6 dargestellt. An seinem proximalen Ende 12 weist der Arbeitskanal 10 eine kreisrunde Querschnittsfläche 16 auf. An seinem distalen Ende 11 weist der Arbeitskanal 10 eine ovale Querschnittsfläche 17 auf. Der ovale Querschnitt erstreckt sich von dem distalen Ende 11 bis zu einem im dem Grundkörper 3 angeordneten Übergangsbereich 18. Von dem Übergangsbereich 18 bis zum proximalen Ende 12 weist der Arbeitskanal 10 einen konstanten kreisrunden Querschnitt auf. Der Übergangsbereich 18 ist konisch ausgebildet.

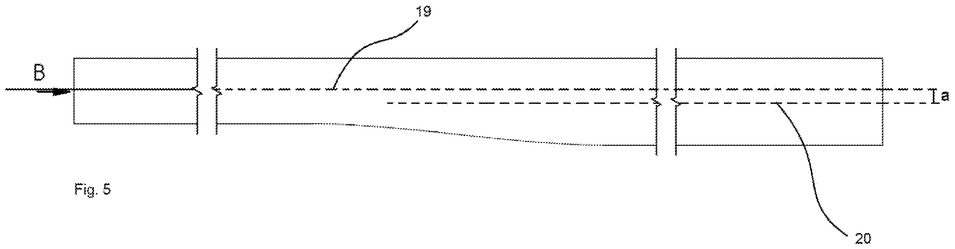

Figur 5 zeigt den Arbeitskanal 10 in einer Seitenansicht. Der Arbeitskanal 10 wird durch ein Rohr gebildet, das mit dem Übergangsbereich 18 bei der Herstellung gezogen wird. Der Arbeitskanal 10 erstreckt sich in seinem Abschnitt mit ovalem Querschnitt entlang einer ersten Arbeitskanal-Längsachse 19. In seinem Abschnitt mit kreisrundem Querschnitt erstreckt sich der Arbeitskanal entlang einer zweiten Arbeitskanal-Längsachse 20. Die beiden Arbeitskanal-Längsachsen 19, 20 sind parallel zueinander. Sie weisen einen Abstand a auf.

In Figur 6 sind die kreisrunde Querschnittsfläche 16 und die ovale Querschnittsfläche 17 im Vergleich dargestellt. Da das den Arbeitskanal 10 bildende Rohr eine gewisse Wandstärke aufweist, sind in Figur 6 zu dem ovalen Querschnitt der innere Querschnitt und der äußere Querschnitt dargestellt. Der Arbeitskanal 10 wird durch den inneren Querschnitt gebildet. Der Durchmesser der kreisrunden Querschnittsfläche 16 ist mit d₁ angegeben. Die ovale Querschnittsflache 17 weist einen größten Durchmesser d₂ und einen kleinsten Durchmesser d₃ auf. Dabei ist d₃ kleiner als d₂. Ferner ist der größte Durchmesser d₂ kleiner als d₁. Die erste Arbeitskanal-Längsachse 19 erstreckt sich durch den Schnittpunkt des größten Durchmessers da mit dem kleinsten Durchmesser d₃ aller ovalen Querschnittsflächen. Die zweite Arbeitskanal-Längsachse 20 erstreckt sich durch den Mittelpunkt aller kreisrunden Querschnittsflächen. Der Abstand a zwischen den beiden Arbeitskanal-Längsachsen 19, 20 ist etwas kleiner als die Differenz aus d₁/2 und d₃/2. Die erste und die zweite Längsachse 19, 20 sind parallel zueinander. Sie verlaufen darüber hinaus parallel zu der Längsachse 4 des Schaftes, welche in Figur 1 dargestellt ist.

Sämtliche Merkmale der Erfindung können sowohl einzeln als auch in beliebiger Kombination miteinander erfindungswesentlich sein.

### Bezugszahlen

- 1: Endoskop
- 2: Schaft
- 3: Grundkörper
- 4: Längsachse
- 5: proximales Ende des Schaftes
- 6: distales Ende des Schaftes
- 7: Bildleiter
- 8: Lichtleiter
- 9: Spülkanal
- 10: Arbeitskanal
- 11: distales Ende des Arbeitskanals
- 12: proximales Ende des Arbeitskanals
- 13: Lichtleiter-Anschluss
- 14: Spülkanal-Anschluss
- 15: Okular
- 16: kreisrunde Querschnittsfläche
- 17: ovale Querschnittsfläche
- 18: Übergangsbereich
- 19: erste Arbeitskanal-Längsachse
- 20: zweite Arbeitskanal-Längsachse

## Patentansprüche

1. Endoskop
mit einem als flexibler oder starrer, länglicher Hohlkörper ausgebildeten Schaft (2),
mit einem distalen Ende (6) und einem proximalen Ende (5) des Schaftes (2),
mit einem Grundkörper (3), in welchem der Schaft (2) mit seinem proximalen Ende (5) aufgenommen ist,
mit einem Bildleiter (7) und einem Arbeitskanal (10), welche sich vom distalen bis zum proximalen Ende durch den Schaft (2) erstrecken und im Grundkörper (3) fortgesetzt sind,
wobei der Arbeitskanal (10) ein Instrument aufnimmt,
mit einem distalen Ende (11) des Arbeitskanals (10), welches sich im Bereich des distalen Endes (6) des Schaftes (2) befindet,
mit einem proximalen Ende (12) des Arbeitskanals (10), welches sich im oder am Grundkörper (3) befindet,
wobei der Querschnitt des Arbeitskanals (10) an seinem proximalen Ende (12) kreisrund und an seinem distalen Ende (11) von kreisrund verschieden ist,
wobei der Arbeitskanal (10) einen Übergangsbereich (18) aufweist, in dem der kreisrunde Querschnitt in den von kreisrund verschiedenen Querschnitt übergeht, **dadurch gekennzeichnet, dass** sich der Übergangsbereich (18) im Grundkörper (3) befindet.

2. Endoskop nach Anspruch 1, **dadurch gekennzeichnet, dass** die Querschnittsfläche des Arbeitskanals (10) am proximalen Ende (12) größer ist als am distalen Ende (11).

3. Endoskop nach Anspruch 1 oder 2, **dadurch gekennzeichnet, dass** der Abschnitt des Arbeitskanals (10), welcher sich außerhalb des Grundkörpers (3) in dem Schaft (2) erstreckt, durchgängig einen von kreisrund verschiedenen Querschnitt mit konstanter Querschnittsfläche aufweist.

4. Endoskop nach einem der vorhergehenden Ansprüche, **dadurch gekennzeichnet, dass** der Arbeitskanal (10) zwischen dem Übergangsbereich (18) und dem proximalen Ende (12) durchgängig einen kreisrunden Querschnitt mit konstanter Querschnittsfläche aufweist.

5. Endoskop nach einem der vorhergehenden Ansprüche, **dadurch gekennzeichnet, dass** der Übergangsbereich (18) im Wesentlichen konisch oder kegelförmig ist.

6. Endoskop nach einem der vorhergehenden Ansprüche, **dadurch gekennzeichnet, dass** die von kreisrund verschiedenen Querschnittsfläche (17) des Arbeitskanals (10) an seinem distalen Ende (11) einen größten Durchmesser und einen kleinsten Durchmesser aufweist, und dass der größte Durchmesser kleiner oder gleich ist wie der Durchmesser der kreisrunden Querschnittsfläche (16) des Arbeitskanals (10) an seinem proximalen Ende (12).

7. Endoskop nach Anspruch 6, **dadurch gekennzeichnet, dass** der Mittelpunkt der von kreisrund verschiedenen Querschnittsfläche (17) des Arbeitskanals (10) an seinem distalen Ende (11) dem Schnittpunkt aus größtem Durchmesser und kleinstem Durchmesser entspricht.

8. Endoskop nach Anspruch 7, **dadurch gekennzeichnet, dass** der Arbeitskanal (10) eine erste Arbeitskanal-Längsachse (19) in seinem Abschnitt mit von kreisrund verschiedenen Querschnitt aufweist, welche sich durch die Mittelpunkte der von kreisrund verschiedenen Querschnittsflächen (17) erstreckt, und dass der Arbeitskanal (10) eine zweite Arbeitskanal-Längsachse (20) in seinem Abschnitt mit kreisrundem Querschnitt aufweist, welche sich durch die Mittelpunkte der kreisrunden Querschnittsflächen (16) erstreckt.

9. Endoskop nach Anspruch 8, **dadurch gekennzeichnet, dass** die Verlängerung der ersten Arbeitskanal-Längsachse (19) mit der zweiten Arbeitskanal-Längsachse (20) zusammenfällt.

10. Endoskop nach Anspruch 8, **dadurch gekennzeichnet, dass** die Verlängerung der ersten Arbeitskanal-Längsachse (19) parallel zu der zweiten Arbeitskanal-Längsachse (20) ist und zu der zweiten Arbeitskanal-Längsachse (20) einen Abstand a aufweist, wobei a von Null verschieden ist.

11. Endoskop nach Anspruch 10, **dadurch gekennzeichnet, dass** der Abstand a kleiner oder gleich der Differenz aus dem halben Durchmesser der kreisrunden Querschnittsfläche (16) des Arbeitskanals (10) an seinem proximalen Ende (12) und dem halben kleinsten Durchmesser der von kreisrund verschiedenen Querschnittsfläche (17) des Arbeitskanals (10) an seinem distalen Ende (11) ist.

12. Endoskop nach einem der vorhergehenden Ansprüche, **dadurch gekennzeichnet, dass** der von kreisrund verschiedene Querschnitt am distalen Ende des Arbeitskanals oval ist.

## Claims

1. Endoscope
with a shaft (2) that is designed as a flexible or rigid elongated hollow body,
with a distal end (6) and a proximal end (5) of the shaft (2),
with a main body (3) which houses the shaft (2) on its proximal end (5),
with an image guide (7) and a working channel (10) which extend through the shaft (2) from the distal to the proximal end, continuing into the main body (3),
wherein the working channel (10) receives an instrument,
with a distal end (11) of the working channel (10) which is located in the area of the distal end (6) of the shaft (2),
with a proximal end (12) of the working channel (10) which is located in or on the main body (3),
wherein the cross-section of the working channel (10) is circular at its proximal end (12) and non-circular at its distal end (11), and
wherein the working channel (10) has a transitional area (18) in which the circular cross-section transitions into the non-circular cross-section, **characterised in that** the transitional area (18) is located in the main body (3).

2. Endoscope according to claim 1, **characterised in that** the cross-section area of the working channel (10) is larger at the proximal end (12) than at the distal end (11).

3. Endoscope according to claim 1 or 2, **characterised in that** the section of the working channel (10) that extends beyond the main body (3) into the shaft (2) has a continuous non-circular cross-section with a constant cross-section area.

4. Endoscope according to one of the previous claims, **characterised in that** the working channel (10) has a continuous circular cross-section with a constant cross-section area between the transitional area (18) and the proximal end (12).

5. Endoscope according to one of the previous claims, **characterised in that** the transitional area (18) is generally conical or tapered.

6. Endoscope according to one of the previous claims, **characterised in that** the non-circular cross-section area (17) of the working channel (10) has a greatest diameter and a smallest diameter at its distal end (11), and that the greatest diameter is smaller than or equal to the diameter of the circular cross-section area (16) of the working channel (10) at its proximal end (12).

7. Endoscope according to claim 6, **characterised in that** the centre point of the non-circular cross-section area (17) of the working channel (10) at its distal end (11) corresponds to the point of intersection from the greatest diameter and the smallest diameter.

8. Endoscope according to claim 7, **characterised in that** the working channel (10) has a first working channel longitudinal axis (19) in its section with non-circular cross-section that extends through the centre points of the non-circular cross-section areas (17), and that the working channel (10) has a second working channel longitudinal axis (20) in its section with circular cross-section that extends through the centre points of the circular cross-section areas (16).

9. Endoscope according to claim 8, **characterised in that** the extension of the first working channel longitudinal axis (19) coincides with the second working channel longitudinal axis (20).

10. Endoscope according to claim 8, **characterised in that** the extension of the first working channel longitudinal axis (19) is parallel to the second working channel longitudinal axis (20) and has a distance 'a' from the second working channel longitudinal axis (20), wherein 'a' is not zero.

11. Endoscope according to claim 10, **characterised in that** the distance 'a' is less than or equal to the difference from half of the diameter of the circular cross-section area (16) of the working channel (10) at its proximal end (12) and half of the smallest diameter of the non-circular cross-section area (17) of the working channel (10) at its distal end (11).

12. Endoscope according to one of the previous claims, **characterised in that** the non-circular cross-section at the distal end of the working channel is oval.

## Revendications

1. Endoscope
avec une tige flexible ou rigide sous forme de corps creux longitudinal (2), avec une extrémité distale (6) et une extrémité proximale (5) de la tige (2), avec un corps de base (3), dans lequel la tige (2) est logée avec son extrémité proximale (5),
avec un guide d'image (7) et un canal de travail (10), qui s'étendent de l'extrémité distale à l'extrémité proximale à travers la tige (2) et jusque dans le corps de base (3),
le canal de travail (10) réceptionnant un instrument,
avec une extrémité distale (11) du canal de travail (10) qui se trouve dans la zone de l'extrémité distale (6) de la tige (2),
avec une extrémité proximale (12) du canal de travail (10) qui se trouve dans ou sur le corps de base (3)
la section du canal de travail (10) à son extrémité proximale (12) étant circulaire et à son extrémité distale (11) autre que circulaire,
le canal de travail (10) présentant une zone de transition (18), dans laquelle la section circulaire passe à la section autre que circulaire,
**caractérisé en ce que**
la zone de transition (18) se trouve dans le corps de base (3).

2. Endoscope selon la revendication 1, **caractérisé en ce que** la surface de section du canal de travail (10) à l'extrémité proximale (12) est plus grande que celle à l'extrémité distale (11).

3. Endoscope selon la revendication 1 ou 2, **caractérisé en ce que** la section du canal de travail (10) qui s'étend en dehors du corps de base (3) dans la tige (2), présente en continu une section autre que circulaire avec une surface constante.

4. Endoscope selon l'une des revendications précédentes, **caractérisé en ce que** le canal de travail (10) entre la zone de transition (18) et l'extrémité proximale (12) présente en continu une section circulaire à surface constante.

5. Endoscope selon l'une des revendications précédentes, **caractérisé en ce que** la zone de transition (18) est principalement conique.

6. Endoscope selon l'une des revendications précédentes, **caractérisé en ce que** la surface de section autre que circulaire (17) du canal de travail (10) à son extrémité distale (11) présente un diamètre maximal et un diamètre minimal et **caractérisé en ce que** le plus grand diamètre est inférieur ou égal au diamètre de la surface de section circulaire (16) du canal de travail (10) à son extrémité proximale (12).

7. Endoscope selon la revendication 6, **caractérisé en ce que** le centre de la surface de section autre que circulaire (17) du canal de travail (10), correspond à son extrémité distale (11) au point d'intersection du plus grand et du plus petit diamètre.

8. Endoscope selon la revendication 7, **caractérisé en ce que** le canal de travail (10) présente un premier axe longitudinal de canal de travail (19) dans son segment à section autre que circulaire, qui passe à travers les centres des surfaces de section autres que circulaires (17) et **caractérisé en ce que** le canal de travail (10) présente un deuxième axe longitudinal de canal de travail (20) dans son segment à section circulaire, qui passe à travers les centres des surfaces de section circulaires (16).

9. Endoscope selon la revendication 8, **caractérisé en ce que** le prolongement du premier axe longitudinal de canal de travail (19) coïncide avec le deuxième axe longitudinal de canal de travail (20).

10. Endoscope selon la revendication 8, **caractérisé en ce que** le prolongement du premier axe longitudinal de canal de travail (19) est parallèle au deuxième axe longitudinal de canal de travail (20) et présente par rapport au deuxième axe longitudinal de canal de travail (20) une distance a, a étant différent de zéro.

11. Endoscope selon la revendication 10, **caractérisé en ce que** la distance a est inférieure ou égale à la différence entre le demi-diamètre de la surface de section circulaire (16) du canal de travail (10) à son extrémité proximale (12) et le plus petit demi-diamètre de la surface de section autre que circulaire (17) du canal de travail (10) à son extrémité distale (11).

12. Endoscope selon l'une des revendications précédentes, **caractérisé en ce que** la section autre que circulaire est ovale à l'extrémité distale du canal de travail.
